(19) Europäisches Patentamt
European Patent Office
Office européen des brevets

(11) Numéro de publication: **0 325 872 B1**

(12) **FASCICULE DE BREVET EUROPEEN**

(45) Date de publication de fascicule du brevet: **26.08.92**

(51) Int. Cl.5: **C12P 19/18**, C07H 3/06, C08B 37/02, A23L 1/236

(21) Numéro de dépôt: **88403084.2**

(22) Date de dépôt: **06.12.88**

(54) **Procédé de préparation enzymatique d'oligodextranes utiles dans la fabrication de substituts du sucre, et nouveaux oligodextranes.**

(30) Priorité: **29.01.88 FR 8801041**

(43) Date de publication de la demande: **02.08.89 Bulletin 89/31**

(45) Mention de la délivrance du brevet: **26.08.92 Bulletin 92/35**

(84) Etats contractants désignés: **AT BE CH DE ES FR GB GR IT LI LU NL SE**

(56) Documents cités:
**EP-A- 0 185 302**
**GB-A- 749 515**
**US-A- 2 726 190**

**CHEMICAL ABSTRACTS, vol. 86, no. 13, 28 mars 1977, page 181, résumé no. 85289z, Columbus, Ohio, US; M.E: PREOBRAZHENSKAYA et al.: "Isolation from dextran of a branched tetrasaccharide, 22-alpha-D-glucosylisomaltotriose, which is resistant to the effect of porcine spleen acid alpha-glucosidase", & DOKL. AKAD. NAUK SSSR 1977, 232(1), 240-3**

(73) Titulaire: **BIOEUROPE**
**4 Impasse Didier Daurat**
**F-31400 Toulouse(FR)**

(72) Inventeur: **Paul, François Bernard**
**28 Rue du Vivier**
**F-31650 Saint-Orens(FR)**
Inventeur: **Lopez Munguia Canales, Agustin**
**Gomez Farias 80-3**
**Coyoacan Mexico 04100 D.F.(MX)**
Inventeur: **Remaud, Magali**
**88 Rue de la Fontaine des Cerdans**
**F-31520 Romonville Saint Agné(FR)**
Inventeur: **Pelenc, Vincent Pascal**
**99 Rue du Férétra**
**F-31400 Toulouse(FR)**
Inventeur: **Monsan, Pierre Frédéric**
**Renoufail Mondonville**
**F-31700 Blagnac(FR)**

(74) Mandataire: **Colas, Jean-Pierre et al**
**Cabinet de Boisse 37, avenue Franklin D. Roosevelt**
**F-75008 Paris(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services

CHEMICAL ABSTRACTS, vol. 88, no. 23, 5 juin 1978, page 457, résumé no. 168503g, Columbus, Ohio, US; && JP-A-77 139 788 (GODO SHUSEI CO., LTD) 21-11-1977

Carbohydrate Res. 121 (1983) 279-286

Carbohydrate Res. 149 (1986) 433-441

## Description

L'invention concerne un procédé de préparation enzymatique d'oligodextranes utiles dans la fabrication de substituts du sucre, ainsi que de nouveaux oligodextranes.

Il est connu par GB-A-749 515 de préparer des dextranes de masse élevée, supérieure au million (degré de polymérisation supérieur à 6000 unités glucose) par l'action de la bactérie lactique Leuconostoc mesenteroides sur du saccharose. On sait aussi, d'après Y. Mitsuishi et coll., Carbohydrate Research, 127 (1984), pages 331-337), que la souche NRRL B-1299 de cette bactérie produit par fermentation des dextranes contenant des liaisons glucosidiques $\alpha$(1 -> 2) qui constituent les points de ramification de ces dextranes, et que ces dextranes peuvent être hydrolysés par voie enzymatique pour produire des oligosaccharides à liaisons $\alpha$(1 -> 2).

Paul et coll. dans Carbohydrate Research, 149 (1986), pages 433-441 ont décrit, de leur côté, la préparation d'oligosaccharides par mise en contact de saccharose avec du maltose en présence de dextranesucrase qui est une glucosyltransférase produite par culture de la souche NRRL B-512F de Leuconostoc mesenteroides. Les oligosaccharides produits ne contiennent pas, toutefois, de liaisons $\alpha$(1 -> 2).

Il n'était pas connu, cependant avant la présente invention, de faire la synthèse enzymatique directe d'oligodextranes (dextranes d'un bas degré de polymérisation) contenant au moins une liaison glucosidique $\alpha$(1 -> 2) à partir de saccharose et d'un sucre accepteur des résidus glucose provenant du saccharose.

La présente invention vise justement à fournir un tel procédé.

Plus précisément, l'invention concerne un procédé de préparation d'oligodextranes contenant une liaison glucosidique $\alpha$(1 -> 2) répondant à la formule générale O-$\alpha$-D-glucopyranosyl-(1 -> 2)) (O-$\alpha$-D-glucopyranosyl-(1 -> 6))$_n$A

où A est le résidu d'un sucre accepteur de glucose choisi parmi le maltose, l'isomaltose, l'isomaltotriose, l'$\alpha$-glucoside de méthyle, et le glucose, n vaut 1, 2 ou 3, la liaison glucosidique $\alpha$(1 -> 2) étant située à l'extrémité non réductrice ou constituant un point de ramification de chaque oligodextrane,

caractérisé en ce qu'on met en contact du saccharose et un sucre accepteur de glucose choisi dans le groupe formé par le maltose, l'isomaltose, l'isomaltotriose, l'$\alpha$-glucoside de méthyle, et le glucose, en présence d'enzyme glucosyltransférase extraite de la souche NRRL B-1299 de la bactérie lactique Leuconostoc mesenteroides, pendant 2 à 48 heures environ, dans un milieu aqueux.

L'invention concerne, aussi, à titre de produits nouveaux, les oligodextranes répondant à la formule générale (O-$\alpha$-D-glucopyranosyl-(1 -> 2)) (O-$\alpha$-D-glucopyranosyl-(1 -> 6))$_n$A où A est un résidu de maltose, n vaut 1, 2 ou 3, la liaison glucosidique $\alpha$(1 -> 2) étant située à l'extrémité non réductrice ou constituant un point de ramification de chaque oligodextrane.

Les oligodextranes produits par le procédé de l'invention qui contiennent une liaison glucosidique $\alpha$(1 -> 2) qui est située à leur extrémité non réductrice ou qui constitue un point de ramification de l'oligodextrane, qu'ils soient nouveaux en eux-mêmes ou non, sont particulièrement résistants à l'hydrolyse enzymatique par des enzymes glucohydrolases, telles que l'endodextranase et la glucoamylase et ceci en raison de la présence de cette liaison glucosidique rare $\alpha$(1 -> 2), située à leur extrémité non réductrice ou constituant un point de ramification de l'oligodextrane.

Cette propriété les rend utiles comme agents de charge ou de corps dans des substituts du sucre peu ou pas métabolisables par l'homme. Ils peuvent donc être utilisés dans des formulations alimentaires pauvres en calories, en mélange avec un agent édulcorant intense, tel que l'aspartame ou équivalent.

Les oligosaccharides de l'invention peuvent également favoriser la croissance et le développement de certains microorganismes bénéfiques de la flore intestinale. Cette propriété peut les rendre utiles aussi bien comme additifs pour l'alimentation animale (additifs d'intérêt zootechnique) que pour l'alimentation humaine (diététique et nutrition).

L'invention concerne donc, en outre, l'utilisation d'oligodextranes contenant une liaison glucosidique $\alpha$(1 -> 2) et répondant à la formule générale (O-$\alpha$-D-glucopyranosyl-(1 -> 2)) (O-$\alpha$-D-glucopyranosyl-(1 -> 6))$_n$A où A est le résidu d'un sucre accepteur de glucose choisi parmi le maltose, l'isomaltose, l'isomaltotriose, l'$\alpha$-glucoside de méthyle, et le glucose, et n vaut 1, 2 ou 3, la liaison glucosidique $\alpha$(1 -> 2) étant située à l'extrémité non réductrice ou constituant un point de ramification de chaque oligodextrane, comme agents de charge dans des substituts du sucre ou comme additifs nutritionnels exerçant un effet bénéfique sur la flore intestinale humaine ou animale.

Comme indiqué ci-dessus, la réaction de synthèse enzymatique est mise en oeuvre en présence d'enzyme glucosyltransférase (E.C:2.4.1.5) extraite de la souche NRRL B-1299 de la bactérie Leuconostoc mesenteroides. Cette réaction peut être représentée par l'équation globale :

```
                          glucosyltransférase
saccharose + sucre accepteur ------> oligodextranes +
fructose
```

La souche NRRL B-1299 est disponible auprès du N.R.R.C. (Northern Regional Research Center) dont l'adresse est la suivante : Agricultural Research Service, U.S. Department of Agriculture, 1815 North University Street, Peoria, Illinois 61604, U.S.A. Elle a été caractérisée au début des années 1950 à partir d'une sélection de microorganismes isolés du sol. Elle a été répertoriée, parmi d'autres, dans la publication suivante:

"Characterization and Classification of dextrans from ninety-six strains of bacteria",

A. Jeanes et al. (1954), J. Amer. Chem. Soc. 76, 5041-5052.

Il va de soi que, au lieu de la souche précitée, on pourrait aussi utiliser des souches dérivant de celle-ci et obtenues par mutagénèse de façon classique, c'est-à-dire par irradiation des souches ou action d'un agent chimique sur lesdites souches, puis culture des individus survivants, ou obtenues par sélection de mutants naturels. Aux fini de l'invention, ces souches mutantes sont considérées comme équivalentes aux souches précitées.

La glucosyltransférase peut être obtenue en cultivant la souche NRRL B-1299 de la bactérie lactique Leuconostoc mesenteroides, sur un milieu nutritif approprié contenant en particulier du saccharose de façon à induire la production de la glucosyltransférase. Après croissance de la bactérie, l'activité enzymatique est extraite par addition de polyéthglèneglycol de façon à précipiter et concentrer les différentes formes de la glucolsyltransférase : extracellulaire, liée aux cellules et aux polysaccharides insolubles, et intracellulaire. Cette préparation enzymatique contient donc les cellules entières de L. mesenteroides B-1299. Des techniques connues de cassage des cellules peuvent être utilisées à la fin de la culture pour augmenter l'activité enzymatique, d'une part, et détruire les cellules, d'autre part (broyage mécanique, emploi d'agents chimiques ou enzymatiques (lysozyme,...). Toutefois, cette opération n'est pas strictement nécessaire. L'extraction de l'activité enzymatique par le polyéthylèneglycol est réalisée une seconde fois après avoir redissous le premier précipité (obtenu après la première extraction), par exemple, à l'aide d'un tampon acétate de sodium 20 mM, pH 5,4 contenant du chlorure de calcium 0,02 g/l. Le deuxième précipité contient toute l'activité enzymatique. Il peut être lyophilisé ou congelé sous forme concentrée sans perte d'activité. D'autres techniques de purification peuvent être utilisées comme la centrifugation, l'ultrafiltration ou un procédé chromatographique.

La synthèse enzymatique d'oligodextranes est réalisée à l'aide de cette préparation enzymatique ou d'une fraction de cette préparation correspondant à une certaine forme de l'enzyme (enzyme intracellulaire, après cassage des cellules, enzyme extracellulaire liée à des polymères insolubles, enzyme extracellulaire soluble) en présence de saccharose qui constitue le substrat de la réaction et d'un sucre connu pour être accepteur de glucose, comme le maltose (ou une matière riche en maltose comme un hydrolysat d'amidon), l'isomaltose, l'$\alpha$-glucoside de méthyle, l'isomaltotriose , le glucose (ou une matière riche en glucose comme un hydrolysat d'amidon).

A titre indicatif, la réaction peut être effectuée entre 5 et 45 ° C, préférentiellement entre 20 et 30 ° C environ. Le pH de la réaction est compris entre 4,5 et 7, de préférence entre 5 et 6. La durée de la réaction est habituellement comprise entre 2 et 48 heures environ ; elle est fonction de la concentration d'enzyme dans le milieu de synthèse. De préférence, la concentration d'enzyme est de 0,20 à 1 U/ml mais pourrait être plus élevée, si désiré. 1 unité enzymatique (U) est définie comme la quantité d'enzyme nécessaire pour produire 1 micromole de fructose par minute dans les conditions standard suivantes de la mesure d'activité :

- saccharose : 100 g/l
- tampon acétate de sodium 20 mM, pH 5,2
- 30 ° C

Les proportions de saccharose et de sucre accepteur de glucose ne sont pas très critiques. A titre indicatif, on peut employer une concentration de saccharose allant de 20 à 600 g/l et une concentration de sucre accepteur de glucose allant de 10 à 300 g/l. Les rendements de synthèse des oligodextranes les plus élevés sont obtenus lorsque le rapport de la concentration de saccharose, exprimée en g/l, à la concentration de l'accepteur, exprimée en g/l, est compris entre 0,5 et 10, de préférence entre 2 et 4.

L'enzyme peut être utilisée soit sous forme libre (procédé discontinu) soit incluse à l'intérieur d'un gel réticulé (gel d'alginate de calcium, par exemple) ou fixée de manière covalente sur un support insoluble. Si l'enzyme est immobilisée, elle peut alors être utilisée dans un réacteur approprié (réacteur à lit fixe, lit fluidisé, etc...) pour la production continue des oligodextranes.

Après action de l'enzyme, le milieu réactionnel peut être analysé par une méthode de chromatographie liquide à haute performance (HPLC) en phase inverse de séparation des oligodextranes (par exemple la colonne micro-Bondapack C18 de Millipore-Waters), l'élution étant réalisée avec de l'eau ultrapure ou un mélange eau/méthanol (% de méthanol compris entre 0 et 6% (v/v)). Cette méthode quantitative permet de séparer tous les oligodextranes de degré de polymérisation compris entre 2 et 20 environ.

Le dosage des sucres réducteurs produits au cours de la réaction peut être réalisé avec le réactif D.N.S. (dinitrosalicylate de sodium en milieu alcalin).

Le mélange réactionnel obtenu comprend des oligodextranes contenant au moins une liaison glucosidique $\alpha$(1 -> 2), des oligodextranes ne comprenant pas une telle liaison, du fructose et du sucre accepteur de glucose inaltéré.

Les oligodextranes à liaison glucosidique $\alpha$(1 -> 2) peuvent constituer de 30 à 55% environ des oligodextranes totaux.

Suivant la masse molaire des oligodextranes recherchée, le procédé de synthèse et purification peut être adapté. En particulier, la masse molaire moyenne des oligodextranes dépend du rapport saccharose/accepteur dans le milieu de synthèse, étant d'autant plus élevée que ce rapport est plus grand. Après synthèse, le fructose peut être conservé dans le milieu réactionnel ou séparé, par une technique chromatographique d'échange d'ions.

Egalement, il faut noter que certains additifs peuvent être ajoutés au milieu de synthèse dans le but d'augmenter la proportion des oligodextranes contenant une liaison $\alpha$(1 -> 2), comme certains solvants miscibles à l'eau tels que des polyéthers comme le monoglyme, le diglyme, etc., ainsi que certains sels tels que le chlorure de magnésium, le chlorure de calcium, etc...

Avantageusement, si l'on désire éliminer les oligodextranes ne contenant pas de liaison glucosidique a-(1 -> 2) du milieu réactionnel, on peut soumettre ce dernier à l'action d'hydrolases comme la glucoamylase d'Aspergillus niger et/ou l'endodextranase de Penicillium sp., afin d'opérer l'hydrolyse totale des oligodextranes ne contenant pas de liaison $\alpha$(1 -> 2). Par contre, les oligodextranes contenant une liaison $\alpha$(1 -> 2), notamment ceux d'un degré de polymérisation de 4, 5, 6 et 7 (en abrégé D.P. 4, D.P.5, D.P.6 et D.P.7) résistent à l'hydrolyse. Après action des hydrolases, le milieu réactionnel contient du glucose, du fructose et les oligodextranes contenant une ou plusieurs liaisons $\alpha$(1 -> 2). Le glucose et le fructose peuvent être séparés, si désiré, des oligodextranes par chromatographie, par exemple à l'aide d'une résine échangeuse cationique sous forme calcium. Les fractions contenant les oligodextranes sont concentrées sous pression réduite, déminéralisées, filtrées sur charbon actif, puis séchées par lyophilisation ou par atomisation. Le produit final a l'aspect d'une poudre blanche très soluble dans l'eau (solubilité : 70%, p/p) non sucrée, de pH neutre, et contient 95% en poids ou plus d'oligodextranes contenant une ou plusieurs liaisons glucosidiques $\alpha$(1 -> 2).

Les exemples non limitatifs suivants sont donnés en vue d'illustrer davantage la présente invention.

EXEMPLE 1

(a) Production et purification de la glucosyltransférase de L. Mesenteroides B-1299

La souche de L. mesenteroides B-1299 est conservée sous forme lyophilisée ou congelée en présence de glycérol à 10% (v/v).

Elle est cultivée dans le milieu de culture suivant (milieu standard) :

- saccharose : 40 g/l
- extrait de levure : 20 g/l
- phosphate dipotassique : 20 g/l (ajouté sous forme d'une solution dont le pH avait été ajusté à 6,9 à l'aide d'acide orthophosphorique pur)
- sulfate de magnésium, $7H_2O$ : 0,2 g/l
- sulfate de manganèse, $H_2O$ : 0,01 g/l
- chlorure de calcium, $2H_2O$ : 0,02 g/l
- chlorure de sodium : 0,01 g/l
- sulfate de fer, $7H_2O$ : 0,01 g/l

Le pH du milieu de culture est égal à 6,9. Le milieu de culture et la solution de phosphate monopotassique ont été préalablement stérilisés par la chaleur (121 °C, 20 minutes). Si le pH n'est pas régulé durant la culture, le milieu s'acidifie au fur et à mesure de la croissance de la bactérie. En fin de culture, le pH peut atteindre 4,5. Il est préférable de réguler le pH à une valeur plus élevée, comprise entre 5 et 6,5. La régulation du pH s'effectue à l'aide de soude 2N ou d'une solution alcaline de saccharose (saccharose 400 g/l ; soude 2N). Cette dernière solution est avantageuse car elle permet d'augmenter

légèrement la densité cellulaire de la culture et la production d'enzyme.

Des additifs, tels que le maltose (20 g/l) et l'agent tensio-actif Tween® (1%) favorisent l'excrétion de l'enzyme dans le milieu extracellulaire sous une forme soluble.

Le tableau 1 ci-après récapitule les résultats de trois essais de culture effectués dans diverses conditions.

TABLEAU 1

| CONDITIONS DE CULTURE | Activité enzymatique totale, U/ml | Activité extracellulaire soluble, U/ml | Concentration d'insolubles (cellules + polysaccharides), mg/ml | Activité insoluble, U/mg |
|---|---|---|---|---|
| essai 1 : Erlenmeyer<br>pas de régulation de pH<br>milieu standard | 3,5 | 0,35 | 7,9 | 0,4 |
| essai 2 : Fermenteur 2 litres<br>milieu standard<br>+ maltose (20 g/l) + Tween® (1%)<br>pas de régulation du pH | 2,75 | 0,6 | 4,3 | 0,5 |
| essai 3 : Fermenteur 2 litres<br>milieu standard<br>+ maltose (20 g/l) + Tween® (1%)<br>+ régulation du pH à 5,6 par addition de saccharose alcalin (saccharose 400 g/l ; soude 2N) | 4,0 | 0,6 | 8,4 | 0,4 |

La température du milieu de culture est de 27° C. L'agitation est de 500 rpm et l'aération égale à 1 vvm.

Après 6 h 30 de culture, la concentration d'enzyme dans le milieu de culture de l'essai 3 est de 4 U/ml, dont 0,6 U/ml de forme enzymatique soluble et extracellulaire. 85% de la glucosyltransférase est associée aux cellules et/ou aux polysaccharides sous forme d'agrégats insolubles.

Après croissance, les différentes formes de l'enzyme sont extraites du milieu de culture par précipitation en présence de polyéthylèneglycol de faible masse molaire (PEG 1500). Toute l'activité enzymatique précipite en même temps que le polysaccharide produit par la bactérie et les cellules, lorsque la concentration de PEG 1500 atteint 20% (p/v).

La fraction précipitée est centrifugée (10 minutes, 10 000 g) ou récupérée après sédimentation (12 heures, 4° C, en présence d'un agent bactériostatique tel que le sulfite de sodium à 1g/litre ou le benzoate de sodium à 2g/litre). Après deux extractions successives par le polyéthylèneglycol, la préparation enzymatique est lyophilisée. Le rendement d'extraction de l'enzyme est voisin de 100%.

(b) Synthèse enzymatique d'oligodextranes avec la glucosyltransférase soluble et insoluble de L. mesenteroides B-1299

La synthèse est réalisée dans les conditions suivantes :

- saccharose : 100 g/l
- maltose : 50 g/l
- température : 30° C
- tampon acétate de sodium 20 mM, pH 5,2
- azoture de sodium : 0,5 ‰ (bactéricide)
- concentration d'enzyme : 0,3 U/ml

Après 20 heures de réaction, l'enzyme est inactivée par la chaleur (30 mn, 80° C) puis les oligodextranes sont analysés par la méthode HPLC précédemment décrite. Les résultats sont rassemblés dans le tableau 2 .

**Tableau 2 : Composition du milieu réactionnel après action de la glucosyltransférase soluble et insoluble de L. mesenteroides B-1299**

| Sucres | Concentration, g/l |
|---|---|
| Fructose | 52,0 |
| Maltose | 15,3 |
| Panose (trisaccharide) | 16,6 |
| Oligodextrane de D.P. 4 | 14,9 |
| Oligodextrane de D.P. 4 contenant une liaison α(1 -> 2) | 5,3 |
| Oligodextrane de D.P. 5 | 7,0 |
| Oligodextrane de D.P. 5 contenant une liaison α(1 -> 2) | 18,0 |
| Oligodextrane de D.P. > 5 contenant au moins une liaison α(1 -> 2) | 5,9 |
| Rendement en oligodextranes contenant une liaison α(1 -> 2)* | 30% |
| Rendement de la réaction d'accepteur ** | 84% |

Les rendements sont exprimés de la manière suivante :

(*)
$$\frac{\text{(oligodextranes } \alpha(1 \to 2)\text{), g/l}}{0{,}474 \times \text{(saccharose)} + \text{(maltose), g/l}}$$

(**)
$$\frac{\text{(oligodextranes totaux), g/l}}{0{,}474 \times \text{(saccharose)} + \text{(accepteur initial-accepteur résiduel), g/l}}$$

(c) Synthèse enzymatique d'oligodextranes avec la glucosyltransférase soluble et insoluble de L. mesenteroides B-1299 en présence d'additifs

Trois synthèses 1, 2 et 3 ont été réalisées dans les conditions communes suivantes :

- saccharose : 100 g/l
- maltose : 33 g/l
- tampon acétate de sodium 20 mM, pH 5,2
- azoture de sodium : 0,5‰
- concentration d'enzyme : 1 U/ml
- température : 30 ° C

mais avec les différences suivantes :

- * synthèse 1 : durée d'incubation : 6 heures (sans additif)
- * synthèse 2 : durée d'incubation : 23 heures, en présence de chlorure de magnésium 500 mM et de chlorure de calcium 5 mM
- * synthèse 3 : durée d'incubation : 23 heures, en présence de chlorure de calcium 300 mM.

Après le temps d'incubation indiqué, l'enzyme est inactivée par la chaleur (30 mn, 80 °C) puis les oligodextranes sont analysés par la méthode HPLC précédemment décrite. Les résultats sont rassemblés dans le tableau 3.

La vitesse de la réaction enzymatique est fortement ralentie en présence de concentrations élevées de sels (chlorure de calcium et chlorure de magnésium). Toutefois, on constate une forte augmentation de la population d'oligodextrares contenant au moins une liaison α(1 -> 2) par rapport à la synthèse témoin (effectuée sans additif).

Tableau 3

| Composition du milieu réactionnel après action de la glucosyltransférase soluble et insoluble de L. mesenteroides B-1299 en présence d'additifs | | | |
|---|---|---|---|
| | Synthèse 1 | Synthèse 2 | Synthèse 3 |
| Fructose | 53,7 | 47,3 | 54,6 |
| Maltose, leucrose | 7,9 | 11,0 | 8,9 |
| Panose | 6,6 | 9,7 | 6,0 |
| Oligodextrane de D.P. 4 | 8,3 | 7,7 | 6,0 |
| Oligodextrane de D.P. 4 contenant une liaison α(1 -> 2) | 1,9 | 5,9 | 3,7 |
| Oligodextrane de D.P. 5 | 5,0 | 5,4 | 4,0 |
| Oligodextrane de D.P. 5 contenant une liaison α(1 -> 2) | 12,2 | 22,8 | 17,9 |
| Oligodextrane de D.P. >5 contenant une liaison α(1 -> 2) | 3,8 | 8,8 | 11,2 |
| Rendement en oligodextranes contenant une liaison α(1 -> 2) | 22% | 47% | 41% |
| Rendement de la réaction d'accepteur | 52% | 75% | 61% |

## EXEMPLE 2

Synthèse d'oligodextranes avec la glucosyltransférase soluble de L. mesenteroides B-1299

Après centrifugation du milieu de culture pour éliminer les cellules et les polymères insolubles (10.000 g, 20 mn), le surnageant est soumis à une extraction liquide-liquide par le polyéthylèneglycol 1.500 selon le procédé décrit dans l'exemple 1. Le rendement d'extraction de la glucosyltransférase est supérieur à 85%. Cette opération a été effectuée deux fois. L'enzyme est alors congelée ou lyophilisée dans du tampon acétate de sodium 20 mM, pH 5,2.

La synthèse est réalisée dans les conditions suivantes :

- saccharose : 100 g/l
- maltose : 50 g/l
- température : 30 °C
- tampon acétate de sodium 20 mM, pH 5,2
- concentration d'enzyme : 0,5 U/ml
- durée de la réaction : 4 heures.

Après consommation totale du saccharose par la glucosyltransférase, l'enzyme est dénaturée par la chaleur (30 minutes, 80 °C). L'analyse des oligodextranes présents dans le milieu réactionnel est réalisée par la méthode HPLC précédemment décrite. Les résultats sont exprimés dans le tableau 4.

Tableau 4

| Composition du milieu réactionnel après action de la glucosyltransférase soluble de L. mesenteroides B-1299 | |
|---|---|
| Sucres | Concentration g/l |
| Fructose | 50,5 |
| Maltose | 11,6 |
| Panose | 17,4 |
| Oligodextrane de D.P. 4 | 19,0 |
| Oligodextrane de D.P. 4 contenant une liaison α(1 -> 2) | 3,1 |
| Oligodextrane de D.P. 5 | 9,9 |
| Oligodextrane de D.P. 5 contenant une liaison α(1 -> 2) | 18,2 |
| Oligodextranes de D.P. > 5 contenant au moins une liaison glucosidique α(1 -> 2) | 13,6 |
| Rendement en oligodextranes contenant au moins une liaison α(1 -> 2) | 36% |
| Rendement de la réaction d'accepteur | 95% |

Les rendements ne prennent pas en compte les oligodextranes de D.P. supérieur ou égal à 7, qui n'apparaissent pas sur les chromatogrammes obtenus par cette méthode.

EXEMPLE 3

Synthèse d'oligodextranes avec la glucosyltransférase insoluble de L. mesenteroides B-1299

Le milieu de culture est centrifugé (20 mn, 10.000 g, 4°C) puis le culot de centrifugation est lavé plusieurs fois à l'aide de tampon acétate de sodium 20 mM pH 5,2. La solution est alors lyophilisée de façon à éviter toute prolifération microbienne.

La synthèse d'oligodextranes est réalisée dans les conditions suivantes :

- saccharose : 100 g/l
- maltose : 50 g/l
- température : 30°C
- tampon acétate de sodium 20 mM, pH 5,2
- azoture de sodium : 0,5 ‰
- concentration d'enzyme : 0,9 U/ml

Après 8 heures d'incubation, le saccharose est totalement consommé. Le Tableau 5 résume la composition des oligodextranes du milieu de synthèse.

Tableau 5

| Composition du milieu réactionnel après action de la glucosyltransférase insoluble (associée aux cellules) de L. mesenteroides B-1299 | |
|---|---|
| Sucres | Concentration g/l |
| Fructose | 51,2 |
| Maltose | 22,0 |
| Panose | 12,8 |
| Oligodextranes de D.P. 4 | 9,6 |
| Oligodextranes de D.P. 4 contenant une liaison α(1 -> 2) | 5,7 |
| Oligodextranes de D.P. 5 | 3,7 |
| Oligodextranes de D.P. 5 contenant une liaison α(1 -> 2) | 13,9 |
| Oligodextranes de D.P. > 5 contenant au moins une liaison α(1 -> 2) | 3,7 |
| Rendement en oligodextranes contenant au moins une liaison α(1 -> 2) | 24% |
| Rendement de la réaction d'accepteur | 65% |

EXEMPLE 4

Influence de la concentration de la glucosyltransférase soluble de L. mesenteroides B-1299 sur la synthèse d'oligodextranes

Conditions expérimentales :

- saccharose : 100 g/l
- maltose : 50 g/l
- tampon acétate de sodium 20 mM, pH 5,2
- température : 30 ° C

Trois concentrations d'enzyme ont été utilisées : 0,17, 0,5 et 2 U/ml. Après consommation totale du saccharose, les 3 milieux réactionnels sont analysés. Les résultats sont identiques pour les trois synthèses aussi bien en ce qui concerne le rendement de la réaction d'accepteur que le rendement en oligodextranes contenant au moins une liaison $\alpha(1 \rightarrow 2)$.

EXEMPLE 5

Influence du pH sur la synthèse enzymatique d'oligodextranes

Conditions expérimentales :

- saccharose : 100 g/l
- maltose : 50 g/l
- température : 30 ° C
- concentration d'enzyme : 1U/ml
- tampon citrate phosphate de sodium 30 mM

Trois pHs ont été testés : 5,2, 6,0 et 6,4. L'activité de l'enzyme soluble et insoluble baisse légèrement lorsque le pH est supérieur à 6,0. Toutefois,, on n'observe pas de différence significative due à la valeur du pH en ce qui concerne la composition en oligodextranes du milieu réactionnel.

EXEMPLE 6

Synthèse d'oligodextranes en présence d'un mélange d'isomaltose et d'isomaltotriose comme accepteur avec la glucosyltransférase insoluble de L. mesenteroides B-1299

Conditions expérimentales

- saccharose : 100 g/l
- accepteur : 50 g/l
  composition de l'accepteur :
  isomaltose : 59%
  isomaltotriose : 36%
  glucose : 5%
- température : 30 ° C
- azoture de sodium : 0,5‰
- tampon acétate de sodium 20 mM, pH 5,2
- concentration d'enzyme : 1 U/ml

Après consommation du saccharose, l'analyse du milieu réactionnel est effectuée à l'aide de la méthode HPLC précédemment décrite (Tableau 6).

Tableau 6

| Composition du milieu réactionnel après action de la glucosyltransférase insoluble de L. mesenteroides B-1299 | |
|---|---|
| Sucres | Concentration, g/l |
| Fructose | 53,0 |
| Isomaltose | 15,0 |
| Isomaltotriose | 6,1 |
| Oligodextranes de D.P. 4 contenant une liaison α(1 -> 2) | 10,4 |
| Isomaltotetraose | 2,4 |
| Oligodextranes de D.P. 5 contenant une liaison α(1 -> 2) | 9,4 |
| Rendement en oligodextranes contenant au moins une liaison α(1 -> 2) | 20,5% |
| Rendement de la réaction d'accepteur | 29,0% |

EXAMPLE 7

Synthèse d'oligodextranes en présence d'α-glucoside de méthyle avec la glucosyltransférase de L. mesenteroides B-1299

Conditions expérimentales :

- saccharose : 100 g/l
- α-glucoside de méthyle : 50 g/l
- autres conditions : voir exemple 6.

Après consommation du saccharose, l'analyse HPLC du milieu réactionnel fournit les résultats suivants (Tableau 7).

Tableau 7

| Composition du milieu réactionnel après action de la glucosyltransférase insoluble de L. mesenteroids B-1299 | |
|---|---|
| Sucres | Concentration, g/l |
| Fructose | 53,0 |
| α-glucoside de méthyle | 34,0 |
| Isomaltoside de méthyle | 4,8 |
| Oligodextranes méthylés contenant une liaison α(1 -> 2) | 9,3 |
| Isomaltotrioside de méthyle | 1,4 |
| Isomaltotetraoside de méthyle | 0,8 |
| Rendement en oligodextranes contenant au moins une liaison α( 1 -> 2) | 10% |
| Rendement de la réaction d'accepteur | 26% |

EXEMPLE 8

Synthèse enzymatique d'oligodextranes en présence de concentrations variables de saccharose avec la glucosyltransférase soluble et insoluble de L. mesenteroides B-1299

Conditions expérimentales :

- température : 30 °C
- tampon acétate de sodium 20 mM, pH 5,2
- azoture de sodium : 0,5‰

- rapport saccharose/maltose : 3

Synthèse 1 :

- matière sèche : 20 % (p/p)
- saccharose : 15% (p/p)
- maltose : 5% (p/p)
- concentration d'enzyme : 0,6 U/ml

Synthèse 2 :

- matière sèche : 35% (p/p)
- saccharose : 26% (p/p)
- maltose : 9% (p/p)
- concentration d/enzyme : 1,2 U/ml

Synthèse 3 :

- matière sèche : 40 % (p/p)
- saccharose : 30% (p/p)
- maltose : 10% (p/p)
- concentration d'enzyme : 1,8 U/ml

Après 21 heures de réaction, l'enzyme est inactivée par la chaleur (30 mn, 80 °C). L'analyse HPLC du milieu réactionnel est reportée dans le tableau 8.

Tableau 8

| Composition des 3 milieux de synthèse après action de la glucosyltransférase soluble et insoluble (associée aux cellules) de L. mesenteroides B-1299 | | | |
|---|---|---|---|
| | Concentration, g/l | | |
| | Synthèse 1 | Synthèse 2 | Synthèse 3 |
| Fructose | 90,0 | 150,0 | 180,0 |
| Maltose | 8,7 | 9,7 | 11,9 |
| Leucrose | 6,3 | 13,9 | 20,4 |
| Panose | 11,7 | 17,5 | 22,9 |
| Oligodextrane D.P.4 | 14,8 | 25,5 | 35,2 |
| Oligodextrane D.P.4 contenant 1 liaison $\alpha$(1 -> 2) | 5,7 | 10,1 | 13,5 |
| Oligodextrane D.P.5 | 10,6 | 17,9 | 20,1 |
| Oligodextrane D.P.5 contenant 1 liaison $\alpha$(1 -> 2) | 28,9 | 44,1 | 53,4 |
| Oligodextranes D.P. $\geqq$ 5 contenant une liaison $\alpha$(1 -> 2) au moins | 15,1 | 36,6 | 46,2 |
| Rendement en oligodextranes contenant une liaison $\alpha$(1 -> 2) | 39% | 40,5% | 38% |
| Rendement de la réaction d'accepteur | 76% | 75% | 70% |

Le milieu de synthèse est ensuite soumis à l'action d'un mélange de glucoamylase d'A. niger (2U/ml) et d'endodextranase de Penicillium sp. (17 U/ml) pendant 6 heures à 40 °C. La réaction enzymatique est stoppée par chauffage à 90 °C pendant 30 minutes. Le glucose et le fructose sont éliminés par chromatographie sur résine échangeuse sous forme calcium.

La concentration d'oligodextranes contenant une liaison glucosidique $\alpha$(1 -> 2) dans le milieu réactionnel est de 57 g/l pour la synthèse 1, de 107 g/l pour la synthèse 2, et de 122 g/l pour la synthèse 3.

La répartition des oligodextranes est la suivante :

| | Synthèse 1 % | Synthèse 2 % | Synthèse 3 % |
|---|---|---|---|
| Oligodextranes de D.P.4 contenant une liaison $\alpha$(1 -> 2) | 24 | 26 | 30 |
| Oligodextranes de D.P.5 contenant une liaison $\alpha$(1 -> 2) | 69 | 65 | 57 |
| Oligodextranes de D.P.6 contenant une liaison $\alpha$(1 -> 2) | 4 | 5 | 7 |
| Oligodextranes de D.P.7 contenant une liaison $\alpha$(1 -> 2) | 3 | 4 | 6 |

## EXEMPLE 9

Synthèse d'oligodextranes en présence d'un sirop de glucose riche en maltose (nutriose R 725) par la glucosyltransférase soluble et insoluble de L. mesenteroides B-1299

Le nutriose R 725 est un produit de la Société Roquette Frères (Lestrem, France), qui contient une teneur élevée en maltose et maltotriose.

Composition moyenne du sirop de glucose Nutriose R 725 :

- matière sèche : 68% (p/p)
- glucose : 1,5%
- maltose : 77 %
- maltotriose : 20%
- produits d'un D.P. ≧ 4 : 1,5%

Ce sirop de glucose a été utilisé comme accepteur dans les conditions suivantes :

Synthèse 1 :

- concentration de saccharose : 100 g/l
- Nutriose R 725 : 68 g/l
- rapport saccharose/maltose : 2
- concentration d'enzyme : 0,3 U/ml
- autres conditions : voir Exemple 8

Synthèse 2 :

- concentration de saccharose : 100 g/l
- Nutriose R 725 : 42 g/l
- rapport saccharose/maltose : 3
- concentration d'enzyme : 0,3 U/ml
- autres conditions : voir Exemple 8

Après 21 heures d'incubation, le milieu réactionnel est analysé par HPLC. Les résultats sont rassemblés dans le Tableau 9.

Tableau 9

| Composition du milieu réactionnel après action de la glucosyltransférase soluble et insoluble de L. mesenteroides B-1299 | | |
|---|---|---|
| | Concentration, g/l | |
| | Synthèse 1 | Synthèse 2 |
| Fructose | 53,0 | 52,0 |
| Maltose | 18,2 | 9,7 |
| Maltotriose | 7,4 | 4,8 |
| Panose | 21,7 | 8,7 |
| Oligodextrane de D.P. 4 | 14,3 | 8,2 |
| Oligodextrane de D.P. 4 contenant une liaison α(1 -> 2) | 4,3 | 2,0 |
| Oligodextrane de D.P. 5 | 4,9 | 4,6 |
| Oligodextrane de D.P. 5 contenant une liaison α(1 -> 2) | 15,0 | 12,9 |
| Oligodextranes de D.P. > 5 (contenant une liaison α(1 -> 2) au moins | 3,8 | 8,5 |
| Rendement en oligodextranes contenant une liaison α(1 -> 2) | 21% | 26% |
| Rendement de la réaction d'accepteurs | 74% | 60% |

Le milieu de synthèse est ensuite soumis à l'action d'un mélange de glucoamylase d'A. niger (2U/ml) et d'endodextranase de Penicillium sp. (17 U/ml) pendant 6 heures à 40° C. La réaction enzymatique est stoppée par chauffage à 90° C pendant 30 minutes. Le glucose et le fructose sont éliminés par chromatographie sur résine échangeuse sous forme calcium.

La concentration d'oligodextranes contenant une liaison glucosidique α(1 -> 2) dans le milieu réactionnel est de 30 g/l pour la synthèse 1 et de 35 g/l pour la synthèse 2.

La répartition des olgodextranes est la suivante :

| | Synthèse 1, % | Synthèse 2, % |
|---|---|---|
| Oligodextranes de D.P. 4 contenant une liaison α(1 -> 2) | 23 | 23 |
| Oligodextranes de D.P. 5 contenant une liaison α(1 -> 2) | 72 | 70 |
| Oligodextranes de D.P. 6 contenant une liaison α(1 -> 2) | 3 | 4 |
| Oligodextranes de D.P. 7 contenant une liaison α(1 -> 2) | 2 | 3 |

## EXEMPLE 10

Hydrolyse des oligodextranes par la glucoamylase d'A. niger ou un mélange de la glucoamylase d/A. niger et de l'endodextranase de Penicillium sp.

Les différents milieux réactionnels des exemples 1 à 9 contiennent des oligodextranes dont le degré de polymérisation varie avec les conditions de réaction. Il est possible d'enrichir le produit en oligodextranes contenant une liaison α(1 2) de D.P. 4 et 5 par action d'hydrolases qui agissent exclusivement sur les liaisons α(1 -> 6) et α(1 -> 4).

La glucoamylase d'A. niger hydrolyse les liaisons α(1 -> 4) et α(1 -> 6) d'oligodextranes à partir de l'extrémité non réductrice. Son action est bloquée par la présence d'une liaison glucosidique α(1 -> 2) quelle que soit sa position sur l'oligodextrane. L'endodextranase hydrolyse exclusivement les liaisons glucosidiques α(1 -> 6) de manière endolytique sur un substrat de degré de polymérisation supérieur ou égal à 3. Toutefois, l'endodextranase n'hydrolyse pas les oligodextranes de D.P. 4 et D.P. 5 qui contiennent une liaison glucosidique α(1 -> 2) à l'extrémité non réductrice. L'action conjuguée de ces deux enzymes permet d'obtenir un produit formé principalement de l'oligodextrane de D.P. 4 et l'oligodextrane de D.P. 5.

Le fructose libéré dans le milieu par action de la glucosyltransférase de L. mesenteroides B-1299 sur le saccharose et le glucose produit par les hydrolases peuvent être séparés simultanément par chromatographie sur résine échangeuse sous forme calcium, technique connue et largement développée industriellement pour la production de sirops à haute teneur en fructose.

Si l'on fait agir la glucoamylase d'A. niger seule, la population d'oligodextranes que l'on obtient

présente un degré de polymérisation moyen plus élevé que dans le cas précédent ; en effet, l'action de la glucoamylase est stoppée à l'extrémité non réductrice de l'oligodextrane en présence d'une liaison glucosidique α(1 -> ->2).

Conditions d'hydrolyse :

- dilution du milieu de synthèse au 1/10ème
- addition d'amyloglucosidase NOVO (300 AGU/ml) : 3 AGU/ml
- addition de dextranase L AMANO (5000 U/ml) : 17 U/ml
- température : 40 ° C
- durée de l'hydrolyse : 6 heures

Après 6 heures d'incubation, les deux enzymes sont inactivées par la chaleur (30 mn, 100 ° C). L'analyse HPLC des deux milieux réactionnels après traitement par les deux hydrolases est reportée dans le Tableau 10.

TABLEAU 10

| | Oligodextranes contenant une liaison α(1 -> 2), D.P. 4 | Oligodextranes contenant une liaison α(1 -> 2), D.P. 5 | Oligodextranes contenant une liaison α(1 -> 2), D.P. > 5 |
|---|---|---|---|
| Accepteur : maltose<br>Conditions de synthèse : voir Exemple 3 | 8 g/l | 17 g/l | 2 g/l |
| Accepteur : isomaltose, isomaltotriose<br>Conditions de synthèse : voir Exemple 6 | 22 g/l | 5 g/l | 1 g/l |

## EXEMPLE 11

Synthèse et purification d'oligodextranes contenant une liaison α(1 -> 2) avec la glucosyltransférase soluble et insoluble de Leuconostoc mesenteroides B-1299

Après avoir préparé et purifié de la glucosyltransférase, comme à l'exemple 1(a), on procède à la synthèse d'oligodextranes dans les conditions suivantes :

- saccharose : 100 g/l
- accepteur : Nutriose R 725 : 44 g/l (maltose : 33 g/l)
- rapport saccharose/maltose : 3
- concentration d'enzyme : 0,3 U/ml
- azoture de sodium : 0,5 ‰
- pH : 5,6 (ajusté avec l'acide chlorhydrique IN)
- température : 30 ° C
- durée d'incubation : 40 heures
- volume réactionnel : 2,5 litres

La réaction enzymatique est stoppée par chauffage à 90 ° C pendant 15 minutes.

Après synthèse, la composition du milieu réactionnel est la suivante :

- fructose : 49 g/l
- leucrose : 6 g/l
- maltose : 3 g/l
- maltotriose : 7,5 g/l
- panose : 12 g/l
- oligodextrane de D.P. 4 contenant une liaison α(1 -> 2) : 3 g/l
- oligodextrane de D.P. 4 : 13 g/l
- oligodextrane de D.P. 5 contenant une liaison α(1 -> 2) : 15 g/l
- oligodextranes de D.P. > 5 contenant une liaison α(1 -> 2) : 10 g/l

Le milieu contient également des oligodextranes de D.P. plus élevé qui n'apparaissent pas dans l'analyse chromatographique du milieu de synthèse.

Le milieu de synthèse est ensuite soumis à l'action d'un mélange de glucoamylase d'A. niger (3U/ml) et d'endodextranase de Penicillium sp. (17 U/ml) pendant 6 heures à 40 ° C. La réaction enzymatique est stoppée par chauffage à 90 ° C pendant 30 minutes. Le glucose et le fructose sont éliminés par chromatographie sur résine échangeuse sous forme calcium.

On obtient ainsi 40 g d'oligodextranes contenant une liaison α(1 -> 2) de pureté égale à 94%. La répartition des oligodextranes est la suivante :

| | % |
|---|---|
| - glucose, leucrose | 6 |
| - oligodextranes de D.P. 4 contenant une liaison α(1 -> 2) | 24 |
| - oligodextranes de D.P. 5 contenant une liaison α(1 -> 2) | 56 |
| - oligodextranes de D.P. 6 contenant une liaison α(1 -> 2) | 7 |
| - oligodextranes de D.P. 7 contenant une liaison α(1 -> 2) | 7 |

La préparation est finalement lyophilisée. Après lyophilisation le produit final se présente sous la forme d'une poudre blanche non hygroscopique, sans saveur sucrée et très soluble dans l'eau. Sa solubilité dans l'eau à 20 ° C est égale à 70% (p/p).

**Revendications**

**1.** Un procédé de préparation d'oligodextranes contenant une liaison glucosidique α(1 -> 2) répondant à la formule générale O-α-D-glucopyranosyl-(1 -> 2)) (O-α-D-glucopyranosyl-(1 -> 6))$_n$A
où A est le résidu d'un sucre accepteur de glucose choisi parmi le maltose, l'isomaltose, l'isomaltotriose, l'α-glucoside de méthyle, et le glucose, n vaut 1, 2 ou 3, la liaison glucosidique α(1 -> 2) étant située α l'extrémité non réductrice ou constituant un point de ramification de chaque oligodextrane,
caractérisé en ce qu'on met en contact du saccharose et un sucre accepteur de glucose choisi dans le groupe formé par le maltose, l'isomaltose, l'isomaltotriose, l'α-glucoside de méthyle, et le glucose, en présence d'enzyme glucosyltransférase extraite de la souche NRRL B-1299 de la bactérie lactique Leuconostoc mesenteroides, pendant 2 à 48 heures environ, dans un milieu aqueux.

**2.** Procédé selon la revendication 1, caractérisé en ce que le pH est maintenu, pendant la réaction enzymatique, entre 4,5 et 6 inclusivement.

3. Procédé selon la revendication 1 ou 2, caractérisé en ce que la réaction enzymatique est conduite à une température de 5 à 45 ° C environ.

4. Procédé selon l'une quelconque des revendications 1 à 3, caractérisé en ce que le rapport en poids saccharose/sucre accepteur de glucose est compris entre 0,5 et 10.

5. Procédé selon la revendication 4, caractérisé en ce que ledit rapport est compris entre 2 et 4 inclusivement.

6. Procédé selon l'une quelconque des revendications 1 à 5, caractérisé en ce que la concentration d'enzyme est comprise entre 0,2 à 1,0 Unité/ml du milieu réactionnel.

7. Procédé selon l'une quelconque des revendications 1 à 6, caractérisé en ce qu'il est mis en oeuvre en présence d'au moins un additif choisi parmi le monoglyme, le diglyme, le chlorure de magnésium et le chlorure de calcium.

8. Procédé selon l'une quelconque des revendications 1 à 7, caractérisé en ce qu'il comprend l'étape supplémentaire consistant à soumettre le milieu réactionnel, après élimination ou inactivation de l'enzyme glucosyltransférase, à l'action d'au moins une enzyme hydrolase audit milieu réactionnel, afin d'hydrolyser sélectivement les oligodextranes dépourvus de liaisons glucosidiques $\alpha$(1 -> 2) présents dans le milieu réactionnel.

9. Procédé selon la revendication 8, caractérisé en ce que l'hydrolase est de la glucoamylase d'Aspergillus Niger et/ou de l'endodextranase de Penicillium sp.

10. Oligodextranes répondant à la formule générale (O-$\alpha$-D-glucopyranosyl-(1 -> 2)) (O-$\alpha$-D-glucopyranosyl(1 -> 6))$_n$A où A est un résidu de maltose, n vaut 1, 2 ou 3, la liaison glucosidique $\alpha$(1 -> 2) étant située à l'extrémité non réductrice ou constituant un point de ramification de chaque oligodextrane.

11. L'utilisation d'oligodextranes contenant une liaison glucosidique $\alpha$(1 -> 2) et répondant à la formule générale (O-$\alpha$-D-glucopyranosyl-(1 -> 2)) (O-$\alpha$-D-glucopyranosyl-(1 -> 6))$_n$A
où A est le résidu d'un sucre accepteur de glucose choisi parmi le maltose, l'isomaltose, l'isomaltotriose, l'$\alpha$-glucoside de méthyle, et le glucose, n vaut 1, 2 ou 3, la liaison glucosidique $\alpha$(1 -> 2) étant située à l'extrémité non réductrice ou constituant un point de ramification de chaque oligodextrane, comme agents de charge dans des substituts du sucre ou comme additifs nutritionnels exerçant un effet bénéfique sur la flore intestinale humaine ou animale.

## Claims

1. A process for the preparation of oligodextrans containing one $\alpha$(1 -> 2) glucoside bond, of the general formula (O-$\alpha$-D-glucopyranosyl-(1 -> 2)) (O-$\alpha$-D-glucopyranosyl-(1-> 6))$_n$A
where A is the residue of a sugar acceptor of glucose chosen from amongst maltose, isomaltose, isomaltotriose, methyl $\alpha$-glucoside and glucose, n is 1, 2 or 3, the $\alpha$(1 -> 2) glucoside bond being located at the non-reducing end or constituting a branching point of each oligodextran, characterized in that sucrose and a sugar acceptor of glucose chosen from the group consisting of maltose, isomaltose, isomaltotriose, methyl $\alpha$-glucoside and glucose are brought into contact in the presence of glucosyltransferase enzyme extracted from the strain NRRL B-1299 of the lactic bacterium Leuconostoc mesenteroides, for approximately 2 to 48 hours, in an aqueous medium.

2. The process as claimed in claim 1, characterized in that the pH is maintained between 4.5 and 6 inclusive during the enzymatic reaction.

3. The process as claimed in claim 1 or 2, characterized in that the enzymatic reaction is carried out at a temperature of about 5 to 45 ° C.

4. The process as claimed in any one of claims 1 to 3, characterized in that the weight ratio of sucrose/sugar acceptor of glucose is between 0.5 and 10.

5. The process as claimed in claim 4, characterized in that said ratio is between 2 and 4 inclusive.

6. The process as claimed in any one of claims 1 to 5, characterized in that the enzyme concentration is between 0.2 and 1.0 units/ml of reaction medium.

7. The process as claimed in any one of claims 1 to 6, characterized in that it is carried out in the presence of at least one additive chosen from amongst monoglyme, diglyme, magnesium chloride and calcium chloride.

8. The process as claimed in any one of claims 1 to 7, characterized in that it includes the supplementary step consisting of subjecting the reaction medium, after removal or inactivation of the glucosyltransferase enzyme, to the action of at least one hydrolase enzyme to the said reaction medium in order to selectively hydrolyse the oligodextrans which do not contain $\alpha$(1 -> 2) glucoside bonds present in the reaction medium.

9. The process as claimed in claim 8, characterized in that the hydrolase is the glucoamylase from Aspergillus Niger and/or the endodextranase from Penicillium sp.

10. The oligodextrans of the general formula (O-$\alpha$-D-glucopyranosyl-(1 -> 2)) (O-$\alpha$-D-glucopyranosyl-(1 -> 6))$_n$A
where A is the residue of maltose, n is 1, 2 or 3, the position of the $\alpha$(1 -> 2) glucoside bond being at the non-reducing end or constituting a branching point of each oligodextran.

11. The use of oligodextrans containing one $\alpha$(1 -> 2) glucoside bond and having the general formula (O-$\alpha$-D-glucopyranosyl-(1 -> 2)) (O-$\alpha$-D-glucopyranosyl-(1-> 6))$_n$A
where A is the residue of a sugar acceptor of glucose chosen from amongst maltose, isomaltose, isomaltotriose, methyl $\alpha$-glucoside and glucose, n is 1, 2 or 3, the $\alpha$(1 -> 2) glucoside bond being at the non-reducing end or constituting a branching point of each oligodextran, as fillers in sugar subtitutes or as foodstuff additives having a beneficial effect on the intestinal flora of humans or animals.

**Patentansprüche**

1. Verfahren zur Herstellung von Oligodextranen, die eine $\alpha$(1 → 2)-glucosidische Bindung enthalten, entsprechend der allgemeinen Formel [O-$\alpha$-D-Glucopyranosyl- (1 → 2)]-[O-$\alpha$-D-glucopyranosyl- (1 → 6)]$_n$A, worin A der Rest eines Glucose-bindenden Zuckers ist, ausgewählt aus Maltose, Isomaltose, Isomaltotriose, $\alpha$-Methylglucosid und Glucose, n den Wert 1, 2 oder 3 hat, die $\alpha$(1 → 2)-glucosidische Bindung sich am nichtreduzierenden Ende befindet oder einen Verzweigungspunkt eines jeden Oligodextrans bildet, dadurch gekennzeichnet, daß Saccharose und ein Glucose-bindender Zucker, ausgewählt aus der Gruppe bestehend aus Maltose, Isomaltose, Isomaltotriose, $\alpha$-Methylglucosid und Glucose, in Gegenwart des Enzyms Glucosyltransferase, gewonnen aus dem Stamm NRRL B-1299 des Lactobakteriums Leuconostoc mesenteroides etwa 2 bis 48 h lang in wäßrigem Milieu zusammengebracht werden.

2. Verfahren nach Anspruch 1, dadurch gekennzeichnet, daß der pH während der enzymatischen Reaktion zwischen 4,5 und 6 gehalten wird.

3. Verfahren nach Anspruch 1 oder 2, dadurch gekennzeichnet, daß die enzymatische Reaktion bei einer Temperatur von etwa 5 bis 45 ° C durchgeführt wird.

4. Verfahren nach irgendeinem der Ansprüche 1 bis 3, dadurch gekennzeichnet, daß das Gewichtsverhältnis von Saccharose/Glucose-bindender Zucker zwischen 0,5 und 10 liegt.

5. Verfahren nach Anspruch 4, dadurch gekennzeichnet, daß dieses Verhältnis zwischen 2 und 4 liegt.

6. Verfahren nach irgendeinem der Ansprüche 1 bis 5, dadurch gekennzeichnet, daß die Enzym-Konzentration zwischen 0,2 bis 1,0 Einheiten/ml Reaktionsmedium liegt.

7. Verfahren nach irgendeinem der Ansprüche 1 bis 6, dadurch gekennzeichnet, daß es in Gegenwart

wenigstens eines Additivs, ausgewählt aus Monoglyme, Diglyme, Magnesiumchlorid und Calciumchlorid, durchgeführt wird.

**8.** Verfahren nach irgendeinem der Ansprüche 1 bis 7, dadurch gekennzeichnet, daß es einen zusätzlichen Schritt umfaßt, der darin besteht, auf das Reaktionsmedium nach Entfernung oder Inaktivierung des Glucosyltransferase-Enzyms wenigstens ein Hydrolase-Enzym einwirken zu lassen, um die im Reaktionsmedium vorhandenen Oligodextrane ohne α(1 → 2)-glucosidische Bindung selektiv zu hydrolysieren.

**9.** Verfahren nach Anspruch 8, dadurch gekennzeichnet, daß die Hydrolase eine Glucoamylase von Aspergillus niger und/oder eine Endodextranase von Penicillium sp. ist.

**10.** Oligodextrane, entsprechend der allgemeinen Formel [O-α-D-Glucopyranosyl- (1 → 2)] [O-α-D-glucopyranosyl-(1 → 6)]$_n$A, worin A der Maltose-Rest ist, n den Wert 1, 2 oder 3 hat, die α(1 → 2)-glucosidische Bindung sich am nichtreduzierenden Ende befindet oder einen Verzweigungspunkt eines jeden Oligodextran bildet.

**11.** Verwendung von Oligodextranen, die eine α(1 → 2)-glucosidische Bindung enthalten und der allgemeinen Formel [O-α-D-Glucopyranosyl- (1 → 2)] [O-α-D-glucopyranosyl-(1 → 6)]$_n$A entsprechen, worin A der Rest eines Glucosebindenden Zuckers ist, ausgewählt aus Maltose, Isomaltose, Isomaltotriose, α-Methylglucosid und Glucose, n den Wert 1, 2 oder 3 hat, die α(1 → 2)-glucosidische Bindung sich am nichtreduzierenden Ende befindet oder einen Verzweigungspunkt eines jeden Oligodextrans bildet, als Füllstoff in Zuckerersatzstoffen oder als Nahrungszusätze, die eine günstige Wirkung auf die Darmflora in Mensch und Tier ausüben.